# EUROPEAN PATENT APPLICATION

(11) **EP 1 486 902 A1**
(43) Date of publication of application: **15.12.2004**
(21) Application number: 04253505.4
(22) Date of filing: 11.06.2004
(51) Int. Cl.: G06F 19/00

(54) **Medical diagnostic apparatus with a display for patient identification on the housing of the apparatus**

(30) Priority: 11.06.2003 JP 2003166035
(71) Applicant: GE Medical Systems Global Technology Company LLC, Waukesha, Wisconsin 53188-1696 (US)
(72) Inventor: Segawa, Koji, Hino-shi, Tokyo 191-8503 (JP); Horiuchi, Tetsuya, Hino-shi, Tokyo 191-8503 (JP)
(74) Representative: Goode, Ian Roy

(57) **Abstract**

For the purpose of reliably preventing misidentification of a patient, a medical diagnostic apparatus comprises: examination means (30) for examining a subject A to generate medical information; operation console means (10) retaining information on an examination plan for a plurality of subjects, for managing the operation of examinations by the examination means (30); and display means (32) provided on a surface of a housing of the examination means (30), for displaying information identifying the current subject to be examined transmitted by the operation console means (10). Thus, the subject (the right patient) A can directly confirm the relationship between himself and the examination means (30) visually from the information identifying the current subject to be examined (e.g., the name of the patient A) displayed on the display means (32) under an inseparable condition.

## Description

The present invention relates to a medical diagnostic apparatus, and more particularly to a medical diagnostic apparatus, such as an X-ray CT apparatus or MRI (magnetic resonance imaging) apparatus, for examining a subject to generate medical information.

In a hospital, examinations are conducted on a large number of medical patients using medical diagnostic apparatuses every day, and it is necessary to accurately identify patients to achieve correct examinations.

In the hospital, a consultation card (e.g., a magnetic card) is created at the reception desk on a patient's first visit, in which card information including the patient ID, name, sex, date of birth, chart number, and name of the department for the patient are recorded. On the next visit, once the patient submits the consultation card at the reception desk, clerical work, such as registration in the consultation queue and retrieval of the patient's chart, proceeds smoothly. A clinician in a certain department examines the patient, and if the clinician judges that X-ray CT imaging is needed, for example, he creates an order sheet for a subsequent examination within the same day or on another day.!

In the radiology department, items in order sheets sent from several departments are input beforehand to a console apparatus for managing examinations, and patients are called up according to the examination plan on that day. A patient whose name is called enters an examination room; however, the conventional method for identifying and confirming a patient only by voice tends to lead to misidentification of a patient having a (phonetically) similar name for the right patient.

When misidentification of a patient occurs, the misidentification is usually discovered at the last examination on that day because a patient whose name was not called is the last one. In another case, misidentification may be noticed from the absence of a disease in the region to be examined when the patient undergoes a scout scan. In any case, X-ray imaging must be done again, leading to not only the patient's discomfort, but also an increase of exposure dose. Therefore, misidentification of a patient must be prevented.

Under such circumstances, there is known a conventional medical examination system for automatically reading a consultation card (IC card) carried by a patient in a non-contact manner, extracting information relating to an examination for the patient by retrieving the identification information of the patient read from the card in a predefined medical database, and displaying the information on a screen of console means operated by a technician etc. (for example, see Japanese Patent Application Laid Open No. 2003-044588). Thus, identification of the right patient is quickly and accurately achieved without the technician orally confirming the patient.

The above system works well if the patient brings the right consultation card, but a senior patient or a patient with multiple diseases, for example, often has a plurality of consultation cards provided by different departments, and if the patient has the wrong consultation card, the patient is eventually misidentified.

Moreover, in an X-ray CT apparatus, the main unit (gantry) of the examination apparatus and the console apparatus for controlling/managing examinations are installed in different rooms, so that a patient confirmed by the technician is not necessarily examined in the right gantry without fail. Especially when a plurality of gantries (CT gantry, angiography/CT apparatus, MRI gantry, etc.) are installed in a room, even if the right patient is called into the room, a mistake may still arise regarding the all-important relationship between the patient and the gantry.

Therefore, an object of the present invention is to provide a medical diagnostic apparatus that can reliably prevent patient misidentification.

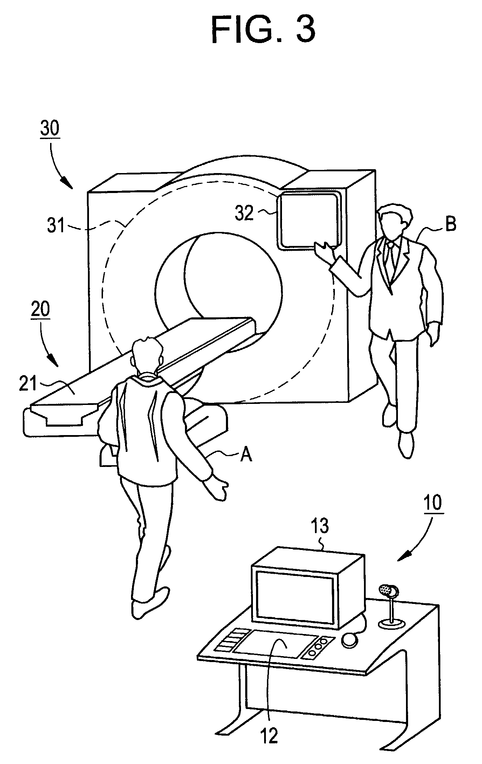

The aforementioned problems are solved by a configuration exemplarily shown in Figure 3. Specifically, a medical diagnostic apparatus in accordance with the present invention (1) comprises: examination means 30 for examining a subject A to generate medical information; operation console means 10 retaining information on an examination plan for a plurality of subjects, for managing the operation of examinations by said examination means 30; and display means 32 provided on a surface of a housing of said examination means 30, for displaying information identifying the current subject to be examined transmitted by said operation console means 10.

According to the present invention (1), the subject (the right patient) A can directly confirm the relationship between himself and the examination means 30 visually from information identifying the current subject to be examined (e.g., the name of the patient A) displayed on the display means 32 on the surface of the examination means 30 under an inseparable condition, and therefore, misidentification of the patient can be reliably prevented.

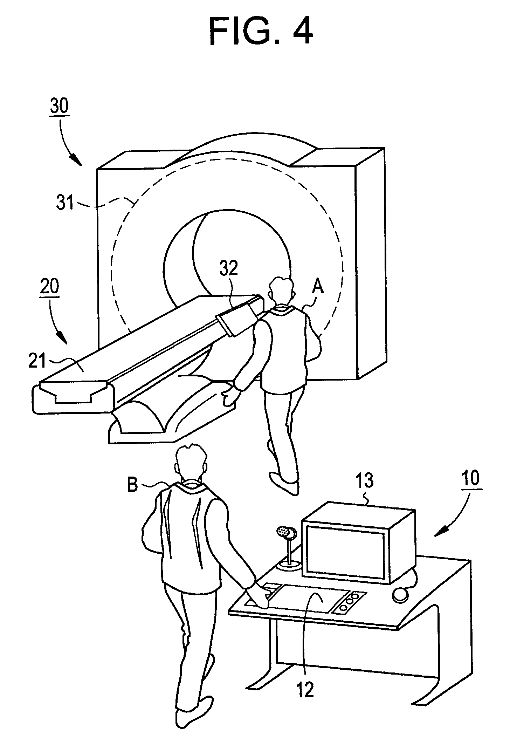

A medical diagnostic apparatus in accordance with the present invention (2), as exemplarily shown in Figure 4, comprises: an imaging table 20 for laying thereon a subject A; examination means 30 for examining the subject laid on said imaging table 20 to generate medical information; operation console means 10 retaining information on an examination plan for a plurality of subjects, for managing the operation of examinations by said examination means 30; and display means 32 provided on a surface of a housing of said imaging table 20, for displaying information identifying the current subject to be examined transmitted by said operation console means 10.

According to the present invention (2), the subject (the right patient) A can indirectly confirm the relationship between himself and the examination means 30 visually, under an inseparable condition, from information identifying a current subject to be examined (e.g., the name of the patient A) displayed on the display means 32 on the surface of the imaging table 20 that is inseparable from the examination means 30, and therefore, misidentification of the patient can be reliably prevented.

In accordance with the present invention (3), in the configuration of the present invention (1) or (2), the information identifying a subject to be examined includes information on the name of the subject. The information on the name of the subject is preferable as information used by the patient to identify himself as the subject to be examined (the right patient).

In accordance with the present invention (4), in the configuration of the present invention (3), the information identifying a subject to be examined further includes information on one of the age, date of birth and sex of the subject or on a combination of two or more of them. By thus additionally displaying the information on the age, date of birth and/or sex of the subject, reliability in identifying the patient is further improved.

In accordance with the present invention (5), in the configuration of the present invention (3), the information identifying a subject to be examined further includes the name of a disease of the subject and/or the name of a region to be examined in the subject. By thus additionally displaying the name of a disease of the subject and/or the name of a region to be examined in the subject, not only misidentification of a patient but also misidentification of a region to be examined can be effectively prevented. Moreover, misidentification of a patient may become noticed from discrepancy of the region to be examined.

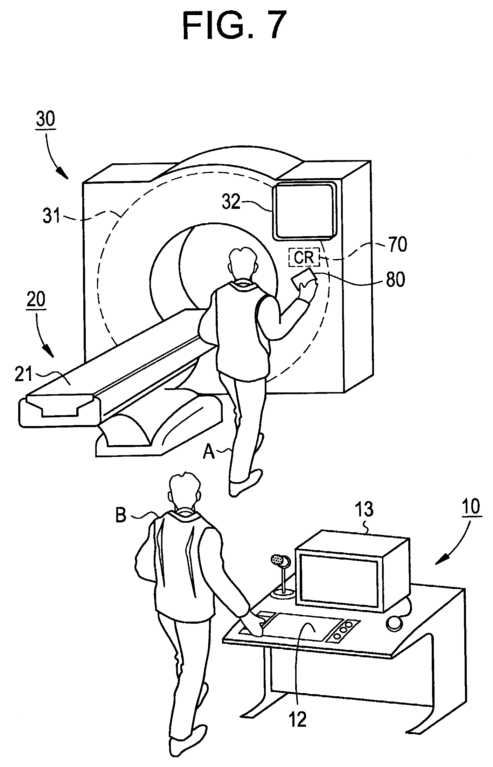

In accordance with the present invention (6), the configuration of the present invention (3) or (5) further comprises information reading means 70 provided near the display means 32 as exemplarily shown in Figure 7, capable of reading in a non-contact manner information identifying a subject to be examined in a portable information carrier 80 that retains the information identifying said subject to be examined, and in such a configuration, the operation console means 10 compares the information identifying the subject to be examined read by the information reading means 70 with information identifying the current subject to be examined managed by said operation console means 10 itself, and when they match, displays information indicating the match on said display means 32.

Various display modes are available for displaying said information indicating the match, such as display of the match by characters, or blinking, inverting or highlighting of a matching portion in the displayed information. Therefore, the patient A himself can easily confirm the match between himself A and the right examination means 30 under an inseparable condition without a technician B or the like orally confirming the patient.

In accordance with the present invention (7), the configuration of the present invention (3) or (5) further comprises information reading means 70 provided near the display means 32 as exemplarily shown in Figure 7, capable of reading in a non-contact manner information identifying a subject to be examined in a portable information carrier 80 that retains the information identifying said subject to be examined, and in such a configuration, the operation console means 10 compares the information identifying the subject to be examined read by the information reading means 70 with information identifying a current subject to be examined managed by said operation console means 10 itself, and when they do not match, displays information indicating the mismatch on said display means 32.

Various display modes are available for displaying said information indicating the mismatch, such as display of the mismatch by characters, or blinking, inverting or highlighting of a mismatching portion in the displayed information. Therefore, the patient A himself can easily confirm the mismatch between himself A and the examination means 30 under an inseparable condition without the technician B or the like orally confirming the patient.

In accordance with the present invention (8), the configuration of the present invention (6) further comprises acoustic output means (not shown) provided near the display means 32 and/or operation console means 10, controlled by the operation console means 10, and in such a configuration, the operation console means 10 outputs a sound indicating the match in conjunction with the display of said information indicating the match.

Various acoustic output modes are available for outputting said sound indicating the match, such as output of a chime sound, or output of a vocal sound (e.g., artificial voice) indicating the match. Therefore, the patient himself and technician can easily confirm the match by the sound, as well as by the display indicating the match.

In accordance with the present invention (9), the configuration of the present invention (7) further comprises acoustic output means (not shown) provided near the display means 32 and/or operation console means 10, controlled by the operation console means 10, and in such a configuration, the operation console means 10 outputs a sound indicating the mismatch in conjunction with the display of said information indicating the mismatch.

Various acoustic output modes are available for outputting the sound indicating the mismatch, such as output of a beep sound, or output of a vocal sound (e.g., artificial voice) indicating the mismatch. Therefore, the patient himself and technician can easily confirm the mismatch by the sound, as well as by the display indicating the mismatch.

In accordance with the present invention (10), in the configuration of the present invention (6), said information indicating the match is displayed by displaying the matching portion of the displayed information in a first predefined color. For example, the matching portion in the displayed information is displayed in blue or green. Therefore, the match can be easily recognized.

In accordance with the present invention (11), in the configuration of the present invention (7), said information indicating the mismatch is displayed by displaying a mismatching portion in the displayed information in a second predefined color. For example, the mismatching portion in the displayed information is displayed in yellow or red. Therefore, the mismatch can be easily recognized.

In accordance with the present invention (12), in the configuration of the present invention (1) ― (11), the examination means is comprised of gantry means for rotating an X-ray imaging system comprising an X-ray tube and an X-ray detector facing each other interposing a subject around the body axis of said subject to acquire projection data. The present invention is suitably applied to an X-ray CT apparatus.

In accordance with the present invention (13), in the configuration of the present invention (1) ― (11), the examination means is comprised of gantry means for detecting a spatial distribution of nuclear magnetization in a subject by a nuclear magnetic resonance technique. The present invention is suitably applied to an MRI apparatus.

Therefore, by using the medical diagnostic apparatus in accordance with the present invention, misidentification of a patient can be reliably prevented, and hence, the contribution of the present invention to improved reliability in medical diagnostic work is very large.

Further objects and advantages of the present invention will be apparent from the following description of the preferred embodiments of the invention as illustrated in the accompanying drawings, in which:
Figure 1 is a diagram showing the configuration of an X-ray CT apparatus in accordance with one embodiment.
Figure 2 is a flow chart of patient identification processing in accordance with a first embodiment.
Figure 3 is a pictorial view (1) for explaining the patient identification operation in accordance with the first embodiment.
Figure 4 is a pictorial view (2) for explaining the patient identification operation in accordance with the first embodiment.
Figure 5 is a diagram showing the configuration of an IC card and a card reader in accordance with one embodiment.
Figure 6 is a flow chart of patient identification processing in accordance with a second embodiment.
Figure 7 is a pictorial view for explaining the patient identification operation in accordance with the second embodiment.

Preferred embodiments of the present invention will now be described in detail with reference to the accompanying drawings. Identical reference symbols designate identical or corresponding portions throughout the drawings.

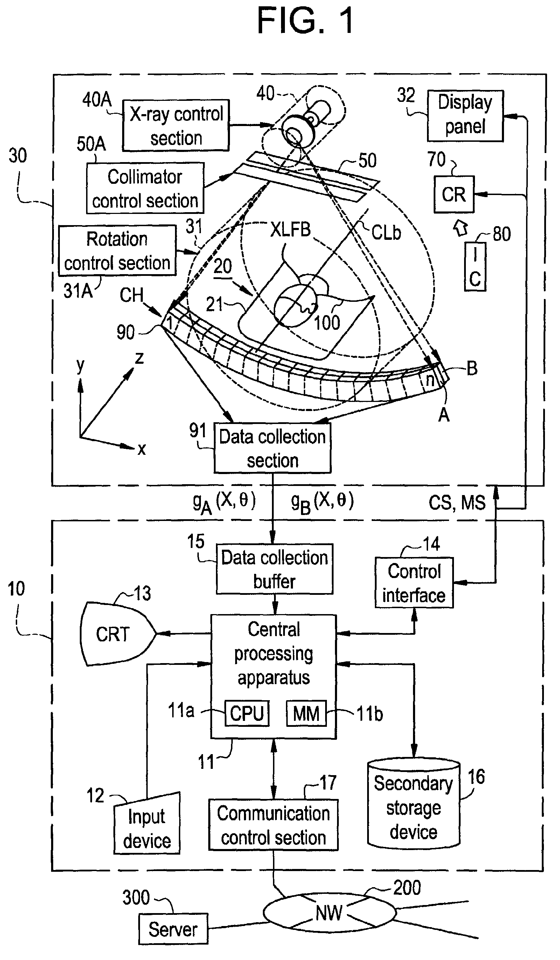

Figure 1 shows the configuration of an X-ray CT apparatus in accordance with one embodiment for explaining an exemplarily application of the present invention to an X-ray CT apparatus. The apparatus is broadly separated into a scan gantry section 30 for conducting an axial/helical scan or the like on a subject 100 using an X-ray fan beam XLFB, an imaging table 20 for laying thereon the subject 100 and carrying the subject 100 in a direction of the body axis CLb, and an operation console section 10 operated by a technician or the like for controlling the sections 30 and 20. The operation console section 10 is generally installed in a neighboring room to protect the technician from radiation accumulation, and the technician can view the examination room from the neighboring room through a windowpane. Although not shown, in this case, the technician can communicate with the patient in the examination room via acoustic equipment (microphones and speakers) provided between the rooms.

In the scan gantry section 30, reference numeral 40 designates a rotary anode X-ray tube, 40A designates an X-ray control section for controlling the tube voltage kV, tube current mA and the like of the X-ray tube, 50 designates a collimator for confining the slice thickness of the X-rays, 50A designates a collimator control section, 90 designates an X-ray detector in which a multiplicity of (n = ca. 1,000) X-ray detector elements lined up in a channel CH direction are arranged in, for example, two rows A and B in the direction of the body axis CLb, 31 designates a scan gantry rotatably supporting the X-ray imaging system as described above around the body axis CLb, 31A designates a rotation control section for the scan gantry 31, 91 designates a data collecting section for generating and collecting projection data of the subject 100 based on signals detected by the X-ray detector 90, 32 designates a display panel for displaying information identifying the subject (the current subject to be examined), 80 designates a consultation card (IC card) carried by the patient, and 70 designates a card reader capable of reading the information in the IC card in a non-contact manner.

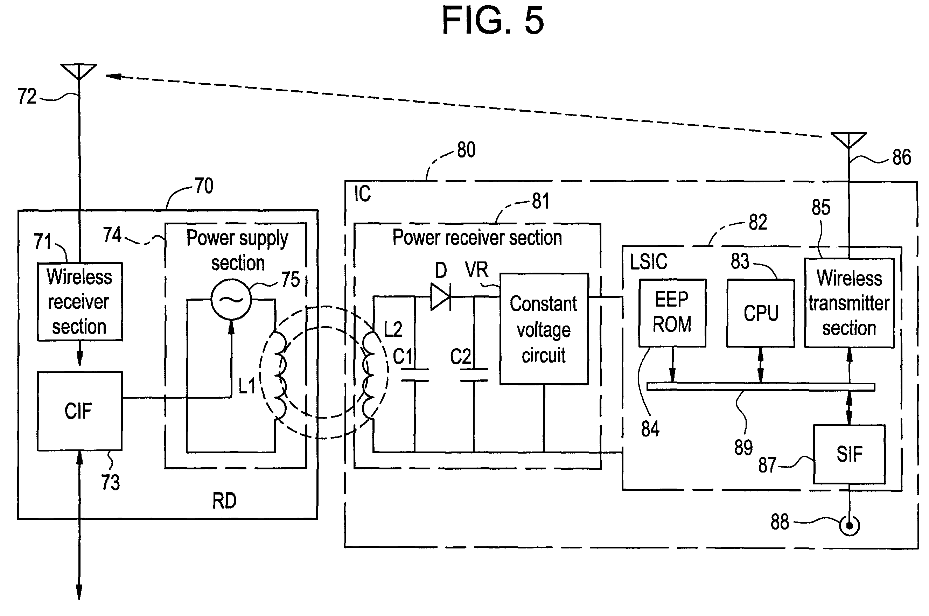

It should be noted that the patient identification process in accordance with the present invention does not necessarily require the card reader 70 and IC card 80, and the patient identification process utilizing them will be described later in a second embodiment with reference to Figures 5 ― 7.

Although not shown, the surface of the housing of the scan gantry section 30 is preferably printed with large characters such as "X-ray CT gantry" so that the patient does not confuse the gantry with an MRI gantry.

In the imaging table 20, reference numeral 21 designates a top plate (cradle) for laying thereon the subject 100 and capable of moving the subject 100 in the direction of the body axis CLb and in the vertical direction. The top plate 21 is also moved up/down when the subject 100 gets on/off the top plate 21.

In the operation console section 10, reference numeral 11 designates a central processing apparatus for conducting main control/processing of the X-ray CT apparatus (scan control, reconstruction of a CT image, processing for assisting subject identification, etc.), 11a designates a CPU of the central processing apparatus 11, 11b designates a main memory (MM) used by the CPU 11a comprised of a RAM, ROM and the like, 12 designates an command/data input device including a keyboard and a mouse, 13 designates a display device (CRT) for displaying information on a scan plan including information identifying the subject (the current subject to be examined), a reconstructed CT image and the like, 14 designates a control interface for exchanging several kinds of control signals CS and monitor signals MS between the CPU 11a and the scan gantry section 30 or imaging table 20, 15 designates a data collection buffer for temporarily storing projection data from the data collecting section 91, 16 designates a secondary storage device (e.g., hard disk device) for storing several kinds of application programs and several kinds of calculation/correction data files necessary for operating the present apparatus, 17 designates a communication control section for connecting to an external network (LAN in a hospital, an intranet, an external internet or the like) 200, and 300 designates a server common to the medical facility for managing/retaining medical information (identification information, chart information etc.) on a large number of patients.

The server 300 stores information identifying a patient and medical information thereof in an area referenced by an ID of that patient. The information identifying a patient includes information on the name, age, date of birth, sex, name of a disease, a region of lesion (an object to be examined) of the patient, and other information characterizing the patient (e.g., the height and weight of the subject and whether the patient uses a wheelchair). The medical information on the patient includes information on an examination order form created by an attendant, chart information of the patient, information relating to the past examination history, and information relating to past X-ray CT examination conditions. Such information is generated on the patient's first visit and also each time the patient visits the hospital thereafter (each time the patient undergoes several kinds of diagnoses), and is stored in the server 300 for each ID of a patient.

In the radiology department, the CPU 11a accesses the server 300 to retrieve information on patients to be examined on the present date, and creates an examination plan. Moreover, when a patient is called according to the created examination plan, the called patient is identified by a process described later, and if the identification is affirmative, the patient is laid on the imaging table 20 and subjected to CT imaging.

Now the operation of the X-ray CT imaging will be briefly explained. An X-ray fan beam XLFB emitted by the X-ray tube 40 passes through the subject 100, and impinges upon the detector rows A and B in the X-ray detector 90 altogether. The data collecting section 91 generates projection data g_{A}(X, θ) and g_{B}(X, θ) corresponding to the outputs from the detector rows in the X-ray detector 90, and stores the data in the data collection buffer 15. In the designation of the projection data, X represents a channel index in the detector, and θ represents a view angle around the body axis CLb. Then, a similar X-ray projection is subsequently conducted at a view angle θ after the scan gantry 31 slightly rotates, and projection data for one rotation of the scan gantry are thus collected and stored. At the same time, the imaging table 20 is moved intermittently or continuously in the direction of the body axis CLb of the subject 100 according to the axial or helical scan scheme, and all projection data for a required imaging region in the subject 100 are thus collected and stored. After the entire scan has been completed, or following (simultaneously with) the scanning, the CPU 11a reconstructs a CT image of the subject 100 based on the resulting projection data, and displays the CT image on the display device 13.

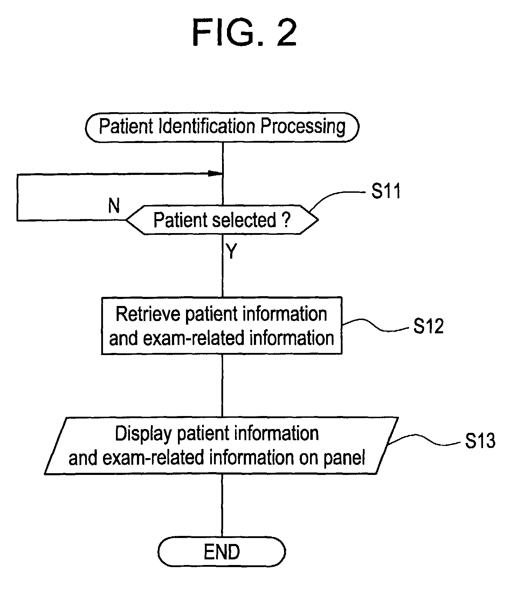

Now the patient identification process in accordance with the present embodiment using the X-ray CT apparatus will be described. Figure 2 is a flow chart of patient identification processing in accordance with the first embodiment, which is executed by the CPU 11a. Figure 3 is a pictorial view (1) for explaining the patient identification operation in accordance with the first embodiment, in which the display panel 32 is provided on a surface of a housing of the scan gantry section 30.

Referring to Figure 2, a selection input for the current subject to be examined (i.e., the patient) is waited for at Step S11, and if patient A, for example, is selected, information identifying the patient A and examination-related information for the patient A are retrieved at Step S12. At Step S13, the retrieved information is displayed on the display panel 32 on the surface of the scan gantry section 30. Moreover, more detailed retrieved information including the retrieved information as described above is also displayed on the display section 13 in the operation console section 10.

Figure 3 shows an exemplary panel display. The display panel 32 consists of an LED panel or back-lit liquid crystal panel, and has a relatively large screen so that it can be easily seen from a distance. The panel 32 is capable of displaying Japanese characters and alphabetic characters, and the patient A can easily confirm his name and the like. In this example, the age (or date of birth) of the patient A and the name of the region to be examined (head, chest, heart, liver, etc.), in addition to the name, are displayed. At that time, the technician B may stand near the operation console section 10 on the other side of the windowpane, but especially when the patient A experiences CT imaging for the first time, the technician B preferably stands near the display panel 32, as shown, to point at the display and vocally prompt the patient A to visually confirm the displayed information. Thus, the patient A can directly confirm the relationship between himself and the CT gantry 30 visually under an inseparable condition. It should be noted that a patient who has experienced the examination several times will spontaneously make visual confirmation without being prompted by the technician.

Figure 4 is a pictorial view (2) for explaining the patient identification operation in accordance with the first embodiment, in which the display panel 32 is provided on a surface of a housing of the imaging table 20. The display panel 32 is provided on a side surface of the imaging table 20 on the side from which the patient gets on/off the imaging table 20, and the patient A can thus easily confirm whether he is to lie on the top plate 21. Moreover, the technician B stands near the operation console section 10 in this case and tells the patient A to confirm his name. Since the imaging table 20 is inseparable from the scan gantry section 30, this method can also reliably prevent misidentification of a patient.

Figure 5 shows the configuration of an IC card and a card reader in one embodiment, in which the card reader is provided adjacent to the display panel 32 to automatically read information identifying a patient in the IC card, and the identification result, i.e., match/mismatch, is displayed on the display panel 32. In Figure 5, reference numeral 70 designates a card reader (CR) for reading information recorded in the IC card 80 in a non-contact (wireless) manner, 71 designates a wireless receiver section according to, for example, a BPSK scheme, 72 designates a receiver antenna of the receiver 71, 73 designates a communication interface (CIF) for connecting to the control interface section 14, 74 designates a power supply section for supply power to the IC card 80 in a non-contact manner, 75 designates a sine wave signal transmitter, and L1 designates an inductor for transmitting power via magnetic coupling.

Moreover, reference numeral 80 designates an IC card, 81 designates a power receiver section for supplying power to the IC card 80, L2 designates a power receiving inductor for receiving supplied power via magnetic coupling with the power transmitting inductor L1, C1 designates a capacitor for resonating at a certain transmitted power frequency, D designates a diode for rectifying the received power, C2 designates a capacitor for smoothing the received power, VR designates a constant voltage regulator for supplying a constant voltage to a load, 82 designates an integrated circuit (LSIC) equipped with several kinds of electronic circuitry, 83 designates a CPU (including a ROM, RAM, etc. for program execution) for conducting main control/processing for the IC card, 84 designates an EEPROM (or flash ROM, etc.) for storing patient identification information in an involatile manner, 85 designates a wireless transmitter section according to the BPSK scheme, for example, 86 designates a transmitter antenna for the wireless transmitter section 85, 87 designates a serial interface (SIF) for writing patient identification information and the like from the external into the EEPROM 84, 88 designates an data input terminal, and 89 designates a common bus for the CPU 83.

When the IC card 80 is newly created, identification information on a patient is written from an external device (not shown) at the reception desk of the hospital into the EEPROM 84 in the IC card 80. The identification information includes a registration ID, the name, date of birth, sex, chart number, name of the department for the patient, and the like. Thereafter, when the patient carries the IC card 80, the LSIC 82 is not supplied with power and the card 80 does not serve as an IC card. However, the information stored in the EEPROM 84 is retained without power supply.

When the patient brings the IC card 80 near the card reader 70, power is supplied from the power supply section 74 to the power receiver section 81 via magnetic coupling between the inductors L1 and L2, and the LSIC 82 is supplied with power. Thus, the CPU 83 is activated and it reads the patient identification information from the EEPROM 84, and then wirelessly transmits the information via the wireless transmitter section 85. Moreover, an electric wave from the antenna 86 is received by the antenna 72, demodulated and subjected to hard decision at the wireless receiver section 71, and the patient identification information data obtained is received by the CPU 11 a via the CIF 73 and control interface 14 in the operation console section 10. When the patient brings the IC card 80 away from the card reader 70, power supply to the LSIC 82 is stopped, whereby the functioning of the LSIC 82 as an IC card is terminated.

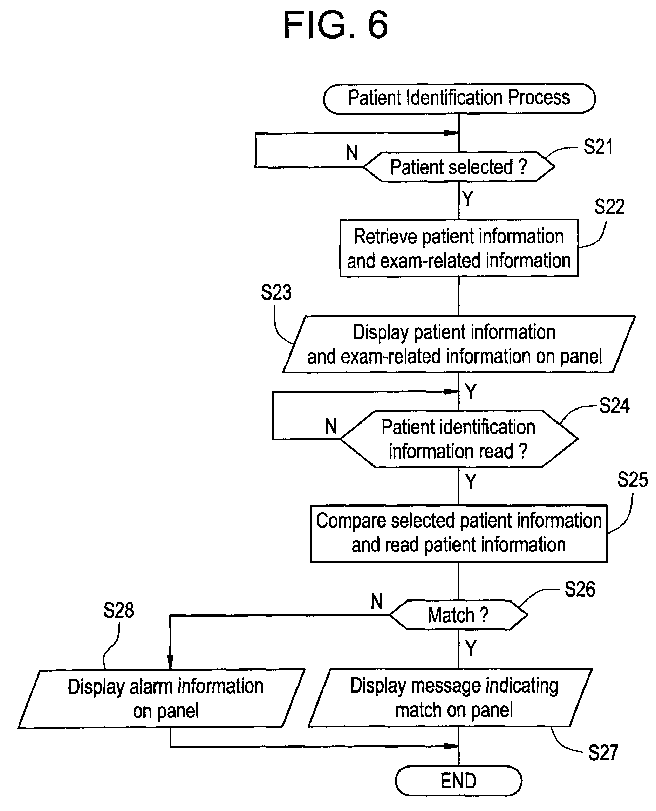

Figure 6 is a flow chart of the patient identification processing in accordance with a second embodiment, in which the patient A whose name is called brings his IC card (consultation card) near a predefined position in the scan gantry section 20 and the patient A can confirm under an inseparable condition match/mismatch between himself and the scan gantry section 20 via the display panel 32. In Figure 6, a selection input for the current subject to be examined (i.e., the patient) is waited for at Step S21, and if patient A, for example, is selected, information identifying the patient A and examination-related information for the patient A is retrieved at Step S22, and then the retrieved information is displayed on the display panel 32 on the surface of the scan gantry section 30 at Step S23. Moreover, more detailed retrieved information including the retrieved information as described above is also displayed on the display section 13 in the operation console section 10.

The technician B calls the patient A based on the displayed information, and waits for reading of the identification information of the patient A from the IC card 80 at Step S24. This state is shown in Figure 7. In this example, the card reader 70 is embedded under the display panel 32 in the scan gantry section 30, and a position toward which the IC card 80 should be brought is marked on the surface of the housing, although not shown. When the patient A entering the examination room brings his IC card 80 near that position, identification information on the patient A is automatically read. At that time, the patient A can in advance visually confirm whether he stands in front of the right CT gantry 30 by looking at the display panel 32.

After the identification information on the patient A has been read, the patient identification information retrieved at Step S22 and the patient identification information read at Step S24 are compared at Step S25, and a decision is made as to whether they match or not at Step S26. If they match, information indicating the match is displayed on the display panel 32 at Step S27, and the patient A can thus confirm that he stands in front of the right CT gantry 30.

Various display modes are available for displaying the information indicating the match, such as display of the match by characters, or blinking, inverting or highlighting of a matching portion in the displayed information. Alternatively, the matching portion in the displayed information may be displayed in color, using a color like blue or green that generally implies affirmation. Therefore, the match can be easily recognized.

If the decision at Step S26 results in mismatch, information indicating the mismatch (alarm information) is displayed on the display panel 32 at Step S28, and the patient A can thus confirm that he stands in front of the wrong CT gantry 30.

Various display modes are available for displaying the information indicating the mismatch, such as display of the mismatch by characters, or blinking, inverting or highlighting of a mismatching portion in the displayed information. Alternatively, the mismatching portion in the displayed information may be displayed in a color like yellow or red that generally implies negation. Moreover, the alarm information at Step S28 may also be displayed on the display section 13 in the operation console section 10 so that the technician B quickly notices the mistake.

Although not shown, it is obvious that the configuration and method of the patient identification according to the second embodiment may be applied to the imaging table 20 shown in Figure 4.

Again, although not shown, acoustic output means controlled by the operation console section 10 may be provided near the display panel 32 and/or operation console section 10, and the operation console section 10 may output a sound indicating the match/mismatch in conjunction with the display of the information indicating the match/mismatch. Various acoustic output modes are available for outputting the sound indicating the match, such as output of a chime sound, or output of a vocal sound (e.g., artificial voice) indicating the match. Various acoustic output modes are available for outputting the sound indicating the mismatch, such as output of a beep sound, or output of a vocal sound (e.g., artificial voice) indicating the mismatch. Therefore, the patient himself and technician can easily confirm the match/mismatch by a sound, as well as by the display indicating the match/mismatch.

Moreover, although the embodiments above address particular examples of application of the present invention to the X-ray CT apparatus, the present invention is not limited thereto. It is obvious that the present invention may be applied to the apparatus, generally called an MRI apparatus, that images a spatial distribution of nuclear magnetization in the subject according to the nuclear magnetic resonance (NMR) method.

## Claims

1. A medical diagnostic apparatus comprising:
an examination device for examining a subject to generate medical information;
an operation console device retaining information on an examination plan for a plurality of subjects, for managing the operation of examinations by said examination device; and
a display device provided on a surface of a housing of said examination device, for displaying information identifying a current subject to be examined transmitted by said operation console device.

2. A medical diagnostic apparatus comprising:
an imaging table for laying thereon a subject;
an examination device for examining the subject laid on said imaging table to generate medical information;
an operation console device retaining information on an examination plan for a plurality of subjects, for managing the operation of examinations by said examination device; and
a display device provided on a surface of a housing of said imaging table, for displaying information identifying a current subject to be examined transmitted by said operation console device.

3. The medical diagnostic apparatus of claim 1 or 2, wherein the information identifying a subject to be examined includes information on the name of the subject.

4. The medical diagnostic apparatus of claim 3, wherein the information identifying a subject to be examined further includes information on one of the age, date of birth and sex of the subject or on a combination of two or more of them.

5. The medical diagnostic apparatus of claim 3, wherein the information identifying a subject to be examined further includes the name of a disease of the subject and/or the name of a region to be examined in the subject.

6. The medical diagnostic apparatus of claim 3 or 5, further comprising:
an information reading device provided near the display device, capable of reading in a non-contact manner information identifying a subject to be examined in a portable information carrier that retains the information identifying said subject to be examined,
wherein said operation console device compares the information identifying the subject to be examined read by the information reading device with information identifying a current subject to be examined managed by said operation console device itself, and when they match, displays information indicating the match on the display device.

7. The medical diagnostic apparatus of claim 3 or 5, further comprising:
an information reading device provided near the display device, capable of reading in a non-contact manner information identifying a subject to be examined in a portable information carrier that retains the information identifying said subject to be examined,
wherein said operation console device compares the information identifying the subject to be examined read by the information reading device with information identifying a current subject to be examined managed by said operation console device itself, and when they do not match, displays information indicating the mismatch on the display device.

8. The medical diagnostic apparatus of claim 6, further comprising:
an acoustic output device provided near the display device and/or operation console device, controlled by the operation console device,
wherein said operation console device outputs a sound indicating the match in conjunction with the display of said information indicating the match.

9. The medical diagnostic apparatus of claim 7, further comprising:
an acoustic output device provided near the display device and/or operation console device, controlled by the operation console device,
wherein said operation console device outputs a sound indicating the mismatch in conjunction with the display of said information indicating the mismatch.

10. The medical diagnostic apparatus of claim 6, wherein said information indicating the match is displayed by displaying a matching portion of the displayed information in a first predefined color.
